(19) Europäisches Patentamt / European Patent Office / Office européen des brevets

(11) **EP 3 191 630 B1**

(12) **EUROPEAN PATENT SPECIFICATION**

(45) Date of publication and mention
of the grant of the patent:
**27.06.2018 Bulletin 2018/26**

(51) Int Cl.:
*C40B 50/06* (2006.01)          *C12Q 1/68* (2018.01)
*C12N 15/10* (2006.01)          *C40B 40/08* (2006.01)

(21) Application number: **14901739.4**

(22) Date of filing: **26.09.2014**

(86) International application number:
**PCT/CN2014/087589**

(87) International publication number:
**WO 2016/037389 (17.03.2016 Gazette 2016/11)**

(54) **METHOD FOR CONSTRUCTING SEQUENCING LIBRARY BASED ON BLOOD SAMPLE AND USAGE THEREOF IN DETERMINING FETAL GENETIC ABNORMALITY**

VERFAHREN ZUM AUFBAU EINER SEQUENZIERUNGSBIBLIOTHEK BASIEREND AUF EINER BLUTPROBE UND VERWENDUNG DAVON BEI DER BESTIMMUNG EINER FÖTALEN GENETISCHEN ANOMALITÄT

PROCÉDÉ DE CONSTRUCTION D'UNE BANQUE DE SÉQUENÇAGE SUR LA BASE D'UN ÉCHANTILLON DE SANG ET UTILISATION DE CELLE-CI POUR LA DÉTERMINATION D'ANOMALIES GÉNÉTIQUES FOETALES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2014 PCT/CN2014/086418**

(43) Date of publication of application:
**19.07.2017 Bulletin 2017/29**

(73) Proprietor: **BGI Genomics Co., Limited**
**Guangdong 518083 (CN)**

(72) Inventors:
• **ZHANG, Yanyan**
  **Shenzhen**
  **Guangdong 518083 (CN)**
• **CHEN, Fang**
  **Shenzhen**
  **Guangdong 518083 (CN)**
• **JIANG, Hui**
  **Shenzhen**
  **Guangdong 518083 (CN)**
• **GUO, Yulai**
  **Shenzhen**
  **Guangdong 518083 (CN)**
• **ZENG, Peng**
  **Shenzhen**
  **Guangdong 518083 (CN)**
• **XU, Xun**
  **Shenzhen**
  **Guangdong 518083 (CN)**
• **YANG, Huanming**
  **Shenzhen**
  **Guangdong 518083 (CN)**
• **BALLINGER, Dennis G.**
  **Menlo Park, CA 94025 (US)**
• **BASHKIROV, Vladimir I.**
  **Davis, CA 95616 (US)**
• **SETHI, Himanshu**
  **Sunnyvale, CA 94085 (US)**

(74) Representative: **HGF Limited**
**8th Floor**
**140 London Wall**
**London EC2Y 5DN (GB)**

(56) References cited:
EP-A1- 2 599 877          WO-A1-2009/061840
WO-A1-2010/030683          WO-A1-2011/074960
WO-A1-2013/059746          WO-A1-2013/112923
WO-A2-2004/070007          WO-A2-2007/140417
WO-A2-2008/015396          WO-A2-2008/070375
WO-A2-2009/116863          WO-A2-2013/191775
US-A1- 2010 069 263          US-A1- 2010 105 052
US-A1- 2011 281 736

• UKAI, HIDEKI ET AL.: 'A new technique to prevent self-ligation of DNA' JOURNAL OF BIOTECHNOLOGY vol. 97, no. 3, 28 August 2002, pages 233 - 242, XP002471622

**Description**

**FIELD**

The present disclosure relates to the biomedical field, particularly to a method for constructing a sequencing library from DNA fragments and usage thereof, more particularly to a method of preparing a library for sequencing based on a blood sample, a method of sequencing a nucleic acid, a method for determining a fetal genetic abnormality which is a chromosomal aneuploidy in a blood sample obtained from a pregnant female subject, an apparatus to prepare a library for sequencing based on a blood sample, an assembly to sequence a nucleic acid, and a system for determining a fetal genetic abnormality which is a chromosomal aneuploidy in a blood sample obtained from a pregnant female subject.

**BACKGROUND**

Chromosome aneuploidy closely relates to some human genetic diseases. Down syndrome is one of the most common chromosome aneuploidies, occurring in 1 in1000, which results from having an extra chromosome 21. Trisomy 13 syndrome and trisomy 18 syndrome are caused by having an extra chromosome 13 and an extra chromosome 18 respectively, with an emergence of miscarriage, and *etc.*. Autosome aneuploidy is another important reason resulting in pregnancy failure and miscarriage. Sex chromosome abnormalities may cause abnormal sexual development. An individual in which males have an extra X chromosome (47, XXY) is Klinefelter syndrome. Turner syndrome, also known as congenital ovarian dysgenesis syndrome, is caused by absence of an entire sex chromosome with a karyotype 45, X.

High throughout sequencing is very useful in the detection of chromosome aneuploidy. However, the means to prepare a library for sequencing still needs to be improved.

**Summary**

The present disclosure is directed to solving at least one of the problems existing in the prior art. For this purpose, a method of preparing a library for sequencing based on a blood sample, a method of sequencing a nucleic acid, a method for determining a fetal genetic abnormality which is a chromosomal aneuploidy in a blood sample obtained from a pregnant female subject, an apparatus to prepare a library for sequencing based on a blood sample, an assembly to sequence a nucleic acid, and a system for determining a fetal genetic abnormality which is a chromosomal aneuploidy in a blood sample obtained from a pregnant female subject are provided.

In one aspect of the present disclosure, a method of preparing a library for sequencing based on a blood sample is provided. According to one aspect of the present invention, a method of preparing a library for sequencing using a DNA sample from a blood samples is thus provided comprising:

(a) isolating a DNA sample from the blood sample,
(b) dephosphorylating the DNA sample to obtain a dephosphorylated fragmentation product, and
(c) subjecting dephosphorylated DNA fragments of said fragmentation product to the following steps to obtain a library of cyclic DNAs for sequencing:

(i) end-repairing to obtain a double-stranded DNA fragment having two blunt ends with four terminal nucleotides each lacking a phosphate group;
(ii) ligating a first adaptor and a second adaptor being different from each other with the double-stranded DNA fragment respectively at the two blunt ends in a one-step reaction to obtain a first ligation product, with each of the first adaptor and the second adaptor being an isolated oligonucleotide comprising: a first strand having a phosphate group at the 5'-terminus and a dideoxynucleotide at the 3'-terminus and a second strand lacking a phosphate group at the 5'-terminus and having a dideoxynucleotide at the 3'-terminus, said first strand having a length greater than that of said second strand and the first and second strands being formed into a double-stranded structure,
(iii) replacing the second strand of the first adaptor with a first single-stranded DNA and replacing the second strand of the second adaptor with a second single-stranded DNA, followed by a nick translation reaction thereby obtaining a second ligation product, with the first single-stranded DNA being adapted to form a double-stranded structure with the first strand of the first adaptor, and the second single-stranded DNA being adapted to form a double-stranded structure with the first strand of the second adaptor,
(iv) amplifying the second ligation product using a first primer and a second primer to obtain an amplified double-stranded DNA fragment, wherein the first primer comprises a sequence identical to one of said first single-stranded DNA and said second single-stranded DNA and the second primer comprises a sequence identical to the other of said first single-stranded DNA and said second single-stranded DNA and possesses an additional biotin;
(v) isolating a single-stranded DNA fragment containing biotin from said amplified double-stranded DNA and
(vi) cyclizing the isolated single-stranded DNA fragment.

The inventors of the present disclosure surprisingly found that because the first and second adaptors each have a dideoxynucleotide at the 3'-terminus nucleotide of the first strand and the double-stranded DNA fragments formed as in step (i) above have two blunt ends with four terminal nucleotides each lacking a phosphate group, ligation between the adaptors and ligation between the double-stranded DNA fragments will not occur. Thus, the efficiency of preparing the library for sequencing will be significantly improved.

According to embodiments of present disclosure, the blood sample is a plasma sample. Furthermore, according to embodiments of the present disclosure, the DNA sample is a circulating nucleic acid sample. The length of the circulating nucleic acid is about 100 to 200 bp, which may be used to prepare the DNA library directly. According to embodiments of the present disclosure, the DNA sample is a genomic DNA sample isolated from a nucleated red blood cell, and the genomic DNA sample is fragmented prior to the step of dephosphorylating to obtain DNA fragments with a length of about 100bp to about 200bp.

According to embodiments of the present disclosure, each of the first and second adaptors independently comprises:

a first overhang end located on the 3'-end of the first strand, and
an optional second overhang end located on the 5'-end of the first strand. The inventors surprisingly found that by using this technical feature, the efficiency of preparing the library for sequencing will be significantly improved.

According to embodiments of the present disclosure, in each of the first and second adaptors, the first overhang end has a length greater than that of the second first overhang end. The inventors surprisingly found that by using this technical feature, the efficiency of preparing the library for sequencing will be significantly improved.

According to embodiments of the present disclosure, in each of the first and second adaptors, the length of the first overhang end is about 6nt to 12nt. The inventors surprisingly found that by using this technical feature, the efficiency of preparing the library for sequencing will be significantly improved.

According to embodiments of the present disclosure, in each of the first and second adaptors, the length of the second overhang end is about 0nt to 4nt. The inventors surprisingly found that by using this technical feature, the efficiency of preparing the library for sequencingwill be significantly improved.

According to embodiments of the present disclosure, in each of the first and second adaptors, the length of the first strand is about 20nt to 25nt. The inventors surprisingly found that by using this technical feature, the efficiency of preparing the library for sequencing will be significantly improved.

According to embodiments of the present disclosure, in each of the first and second adaptors, the length of the second strand is about 10nt to 15nt. The inventors surprisingly found that by using this technical feature, the efficiency of preparing the library for sequencing will be significantly improved.

As noted above, the step of ligating the first adaptor and the second adaptor with double-stranded DNA fragments is performed in a one-step reaction. The inventors surprisingly found that by using this technical feature, the efficiency of preparing the library for sequencing will be significantly improved.

According to embodiments of the present disclosure, the double-stranded structure formed by the first single-stranded DNA and the first strand of the first adaptor has a length greater than that of the double-stranded structure formed by the first strand and the second strand in the first adaptor, and the double-stranded structure formed by the second single-stranded DNA and the first strand of the second adaptor has a length greater than that of the double-stranded structure formed by the first strand and the second strand in the second adaptor. The inventors surprisingly found that by using this technical feature, the efficiency of preparing the library for sequencing will be significantly improved.

According to embodiments of the present disclosure, the first strand of the first adaptor comprises a sequence of 5' AAGTCGGAGGCCAAGCGGTCGT 3',
the second strand of the first adaptor comprises a sequence of 5' TTGGCCTCCGACT 3',
the first strand of the second adaptor comprises a sequence of 5' GTCTCCAGTCGAAGCCCGACG 3',
the second strand of the second adaptor comprises a sequence of 5' GCTTCGACTGGAGA 3',
the first single-stranded strand DNA comprises a sequence of 5'TCCTAAGACCGCTTGGCCTCCG3', and the second single-stranded DNA comprises a sequence of 5'AGACAAGCTC(N)$_m$GATCGGGCTTCGACGGAG3', wherein motif (N)$_m$ represents an index sequence comprising m nucleotides, m is any integer ranging from 4nt to 10nt, N=A, T, G or C, and
the single-stranded DNA employed for cyclization comprises a sequence of 5' TCGAGCTTGTCTTCCTAAGACCGC 3'. The inventors surprisingly found that by using this technical feature, the efficiency of preparing the library for sequencing will be significantly improved.

According to embodiments of the present disclosure, the step of replacing the second strand of the first adaptor with the first single-stranded DNA and replacing the second strand of the second adaptor with the second single-stranded DNA is performed by means of thermal lysis and annealing. The inventors surprisingly found that by using this technical feature, the efficiency of preparing the library for sequencing will be significantly improved.

Furthermore, according to embodiments of the present disclosure, the thermal lysis is performed at a temperature of about 60 degrees Celsius. The inventors surprisingly found that by using this technical feature, the efficiency of preparing the library for sequencing will be significantly improved.

As noted above, the step of ligating the first single-stranded DNA and the second single-stranded DNA with the double-stranded DNA respectively at the two blunt ends is performed by means of a nick translation reaction. The inventors surprisingly found that by using this technical feature, the efficiency of preparing the library for sequencing will be significantly improved.

According to embodiments of the present disclosure, the step of isolating the single-stranded DNA fragment containing biotin from the amplified double-stranded DNA fragment further comprises the steps of:

contacting the amplified double-stranded DNA fragment with a magnetic bead to form a bead-DNA composite, with the magnetic bead having a streptavidin linked thereto, and

contacting the bead-DNA composite with a solution having a pH greater than 7 to obtain the single-stranded DNA fragment containing biotin. The inventors surprisingly found that by using this technical feature, the efficiency of preparing the library for sequencing will be significantly improved.

Furthermore, according to embodiments of the present disclosure, the solution having a pH greater than 7 comprises NaOH. The inventors surprisingly found that by using this technical feature, the efficiency of preparing the library for sequencing will be significantly improved.

According to embodiments of the present disclosure, the concentration of NaOH in the solution is about 0.5M to about 2M. The inventors surprisingly found that by using this technical feature, the efficiency of preparing the library for sequencing will be significantly improved.

Furthermore, according to embodiments of the present disclosure, the concentration of NaOH in the solution is about 1M. The inventors surprisingly found that by using this technical feature, the efficiency of preparing the library for sequencing will be significantly improved.

A method of the invention may further comprise a step of screening the amplified double-stranded DNA fragment prior to the step of isolating the single-stranded DNA fragment containing the biotin. The inventors surprisingly found that by using this technical feature, the efficiency of preparing the library for sequencing will be significantly improved.

Furthermore, according to embodiments of the present disclosure, the screening is performed by contacting the amplified double- stranded DNA fragment with a probe specific to a predetermined sequence. The inventors surprisingly found that by using this technical feature, the efficiency of preparing the library for sequencing will be significantly improved.

According to embodiments of the present disclosure, the predetermined sequence comprises at least one exon. The inventors surprisingly found that by using this technical feature, the efficiency of preparing the library for sequencing will be significantly improved.

The probe may be provided in the form of a microarray. The inventors surprisingly found that by using this technical feature, the efficiency of preparing the library for sequencing will be significantly improved.

According to embodiments of the present disclosure, the step of cyclizing the isolated single-stranded DNA fragment containing biotin is performed by using a single-stranded nucleic acid molecule comprising:

a first section adapted to match with either one of a sequence comprising the 5'-terminus nucleotide or a sequence comprising the 3'-terminus nucleotide in said isolated single-stranded DNA fragment containing biotin, and

a second section adapted to match with the other of a sequence comprising the 5'-terminus nucleotide or the 3'-terminus nucleotide in said isolated single-stranded DNA fragment containing biotin. The inventors surprisingly found that by using this technical feature, the efficiency of preparing the library for sequencing will be significantly improved.

According to embodiments of the present disclosure, the first section and the second section are adjacently linked together, for example, the single-stranded nucleic acid employed for cyclization comprises the sequence 5' TCGAGCTTGTCTTCCTAAGACCGC 3' as discussed above. The inventors surprisingly found that by using this technical feature, the efficiency of preparing the library for sequencing will be significantly improved.

In another aspect of the present disclosure, there is provided a method of sequencing a nucleic acid, comprising the steps of:

preparing a library for sequencing containing said nucleic acid according to a method of the invention as described above, and

sequencing the library.

According to embodiments of the present disclosure, the step of sequencing is performed by using a CG sequencing platform.

In another aspect of the present disclosure, there is provided a method for determining a fetal genetic abnormality which is a chromosomal aneuploidy in a blood sample obtained from a pregnant female subject, comprising:

(a) sequencing at least a portion of a plurality of the nucleic acid molecules contained in the blood sample to obtain a sequencing result according to the method described above;

(b) aligning the sequencing result obtained in step (a) with a reference human genome sequence to obtain an aligned reads group;

(c) determining the total number of reads contained in the aligned reads group obtained in step (b), to obtain a value of M;

(d) based on the aligned reads group obtained in step (b), determining the number of reads originating from a chromosome i, to obtain a value of $N_i$, wherein i represent a chromosome number;

(e) based on the values M and $N_i$, determining a parameter representing relative percentage of chromosome i, to obtain a parameter R; and

(f) based on the parameter R, determining whether a fetal genetic abnormality is present for the chromosome i.

Using the above method, a chromosomal aneuploidy may be effectively detected.

According to embodiments of present disclosure, the read has a length ranging from about 12bp to 21bp.

According to embodiments of the present disclosure, the aligned reads group consists of uniquely aligned reads. The inventors surprisingly found that by using this technical feature, the efficiency of detecting a chromosomal aneuploidy will be significantly improved.

According to embodiments of the present disclosure, in step (f), the parameter R is the ratio of $N_i$/M. The inventors surprisingly found that by using this technical feature, the efficiency of detecting a chromosomal aneuploidy will be significantly improved.

According to embodiments of present disclosure, step (f) further comprises:

obtaining a Z value based on the equation of

$$Z_i = (R_{i,j} - mean_i)/sd_i,$$

wherein,

$$mean_i = \frac{1}{n}\sum_{j=1}^{n} R_{i,j},$$

$$sd_i = \sqrt{\frac{1}{n-1}(R_{i,j} - mean_i)^2}$$

j represents sample number, n represents the total number of the samples; and

comparing the Z value with a first preset value and a second preset value. The inventors surprisingly found that by using this technical feature, the efficiency of detecting a chromosomal aneuploidy will be significantly improved.

According to embodiments of the present disclosure, the first preset value and second preset value are determined using a plurality of known samples. The inventors surprisingly found that by using this technical feature, the efficiency of detecting a chromosomal aneuploidy will be significantly improved.

According to embodiments of the present disclosure, the first preset value is no more than -3. The inventors surprisingly found that by using this technical feature, the efficiency of detecting a chromosomal aneuploidy will be significantly improved.

According to embodiments of the present disclosure, the second preset value is at least +3. The inventors surprisingly found that by using this technical feature, the efficiency of detecting a chromosomal aneuploidy will be significantly improved.

According to embodiments of the present disclosure, a Z value of less than the first preset value is an indication of the presence of an additional chromosome i in the sample, and a Z value of greater than the second preset value is an indication of the absence of one chromosome i in the sample. The inventors surprisingly found that by using this technical feature, the efficiency of detecting a chromosomal aneuploidy will be significantly improved.

The present disclosure also provides an apparatus to perform the above-described method.

In another aspect of the present disclosure, there is provided an apparatus to prepare a library for sequencing based on a blood sample and in accordance with the invention, comprising:

(a) a DNA-sample-isolating unit adapted to isolate a DNA sample from the blood sample;

(b) a dephosphorylation unit adapted to dephosphorylate the DNA sample to obtain a dephosphorylated fragmentation product;

(c) an end-repairing unit adapted to end-repair the dephosphorylated fragmentation product to obtain a double-stranded DNA fragment, with the double-stranded DNA fragment having two blunt ends with four terminal nucleotides each lacking phosphate group;

(d) a first ligation unit adapted to ligate a first adaptor and a second adaptor being different from each other with the double-stranded DNA fragment respectively at the two blunt ends, to obtain a first ligation product, with each of the first adaptor and the second adaptor being an isolated oligonucleotide comprising: a first strand having a phosphate group at the 5'-terminus and a dideoxynucleotide at the 3'-terminus nucleotide and a second strand lacking a phosphate group at the 5'-terminus and having a dideoxynucleotide at the 3'-terminus nucleotide, said first strand having a length greater than that of said second strand and the first and second strands being formed into a double-stranded structure,

(e) a strand-replacing and nick translation unit adapted to replace the second strand of the first adaptor with a first single-stranded DNA and replace the second strand of the second adaptor with a second single-stranded DNA followed by a nick translation reaction thereby obtaining a second ligation product with the first single-stranded DNA being adapted to form a double-stranded structure with the first strand of the first adaptor, and the second single-stranded DNA being adapted to form a double-stranded structure with the first strand of the second adaptor,

(f) an amplification unit adapted to amplify the second ligation product using a first primer and a second primer to obtain an amplification product, wherein the first primer comprises a sequence identical to one of said first single-stranded DNA and said second single-stranded DNA and the second primer comprises a sequence identical to the other of said first single-stranded DNA and said second single-stranded DNA and possesses an additional biotin and the amplification product is an amplified double-stranded DNA fragment;

(g) a single-strand-DNA-isolating unit adapted to isolate a single-stranded DNA fragment containing the biotin from the amplified double-stranded DNA fragment and

(h) a cyclizing unit adapted to cyclize the isolated single-strand DNA fragment whereby a library of cyclic DNAs can be formed for sequencing.

The inventors surprisingly found that by using this technical feature, the efficiency of detecting a chromosomal aneuploidy will be significantly improved. The strand-replacing unit is adapted to replace the second strand of the first adaptor with the first single-stranded DNA and replace the second strand of the second adaptor with the second single-stranded DNA by means of thermal lysis and annealing.

According to embodiments of the present disclosure, the singe-strand-DNA-isolating unit is adapted to perform the steps of:

contacting the amplified double-stranded DNA fragment with a magnetic bead to form a bead-DNA composite, with the magnetic bead having a streptavidin linked thereto, and

contacting the bead-DNA composite with a solution having a pH greater than 7 to obtain the single-stranded DNA fragment containing biotin.

According to embodiments of the present disclosure, the blood sample is a plasma sample, optionally, the DNA sample is a circulating nucleic acid sample. According to embodiments of the present disclosure, the DNA sample is a genomic DNA sample isolated from a nucleated red blood cell and the apparatus further comprises a fragmentation unit adapted to fragment the DNA to obtain a DNA fragments with a length of about 100bp to about 200bp.

An apparatus according to the invention as described above may further comprise a screening unit adapted to perform a step of screening the amplified double-stranded DNA fragment prior to the step of isolating the single-stranded DNA fragment containing the biotin.

According to embodiments of the present disclosure, the screening is performed by contacting the amplified double-stranded DNA fragment with a probe specific to a predetermined sequence.

According to embodiments of the present disclosure, the cyclizing unit is adapted to cyclize the isolated single-stranded DNA fragment by using a single-stranded nucleic acid molecule, comprising:

a first section adapted to match with a sequence comprising the 5'-terminus nucleotide or a sequence comprising the 3'-terminus nucleotide in said isolated single-stranded DNA fragment containing biotin, and a second section adapted to match with the other of sequence comprising the 5'-terminus nucleotide or the 3'-terminus nucleotide in said isolated single-stranded DNA fragment containing biotin.

In another aspect of the present disclosure, there is provided an assembly to sequence a nucleic acid, comprising:

an apparatus to prepare a library for sequencing based on a blood sample as described above, and a sequencing apparatus adapted to sequence the library.

According to embodiments of the present disclosure, the sequencing apparatus is a CG sequencing platform.
In another aspect of the present disclosure, there is provided a system for determining a fetal genetic abnormality which is a chromosomal aneuploidy in a blood sample obtained from a pregnant female subject, comprising:

an above-mentioned assembly adapted to sequence at least a portion of a plurality of the nucleic acid molecules contained in the blood sample to obtain a sequencing result; an assembly adapted to analyse the sequencing result according to the following steps of:

aligning the sequencing result with a reference human genome sequence to obtain an aligned reads group; determining the total number of reads contained in the aligned reads group to obtain a value of M; based on the aligned reads group, determining the number of reads originating from a chromosome i, to obtain a value of $N_i$, wherein i represent a chromosome number; based on the values M and $N_i$, determining a parameter representing relative percentage of chromosome i, to obtain a parameter R; and based on the parameter R, determining whether a fetal genetic abnormality is present for the chromosome i.

According to embodiments of the present disclosure, the read has a length ranging from about 12 bp to 21 bp.
According to embodiments of the present disclosure, the aligned reads group consists of uniquely aligned reads.
According to embodiments of the present disclosure, the parameter R is the ratio of $N_i/M$. According to embodiments of the present disclosure, the steps further comprise:
obtaining a Z value based on equation of

$$Z_i = (R_{i,j} - mean_i) / sd_i,$$

wherein,

$$mean_i = \frac{1}{n} \sum_{j=1}^{n} R_{i,j},$$

$$sd_i = \sqrt{\frac{1}{n-1}(R_{i,j} - mean_i)^2}$$

j represents sample number, n represents the total number of the samples; and comparing the Z value with a first preset value and a second preset value.

According to embodiments of the present disclosure, the first preset value and second preset value are determined using a plurality of known samples.
According to embodiments of the present disclosure, the first preset value is no more than -3. According to embodiments of the present disclosure, the second preset value is at least +3.
According to embodiments of the present disclosure, a Z value of less than the first preset value is an indication of the presence of an additional chromosome i in the sample, and a Z value of greater than the second preset value is an indication of the absence of one chromosome i in the sample.

The technical solutions of the present disclosure may achieve the following advantages:
The fetal genetic abnormality of a chromosomal aneuploidy may be detected using blood samples, for example a 1ml blood sample, even at only 9 gestational weeks. Moreover, according to embodiments of the present disclosure, about 3000 to about 5000 samples may be analyzed at one time.

Additional aspects and advantages of embodiments of the present disclosure will be given in part in the following descriptions, become apparent in part from the following descriptions, or be learned from the practice of embodiments of the present disclosure.

## DETAILED DESCRIPTION

Reference will be made in detail to embodiments of the present disclosure. The embodiments described herein with reference to the drawings are explanatory, illustrative, and used to generally understand the present disclosure. The embodiments shall not be construed to limit the present disclosure.

In addition, terms such as "first" and "second" are used herein for purposes of description and are not intended to indicate or imply relative importance or significance. Thus, features restricted with "first", "second" may explicitly or implicitly comprise one or more of the features. Furthermore, in the description of the present disclosure, unless otherwise stated, the term "a plurality of" refers to two or more. A term "aneuploidy" used herein is a relative term with euploidy of a chromosome, which refers to one or more chromosome missing or extra in the genome. In general, there are two chromosomes of each type in normal cells. However, a gamete having an abnormal number of chromosomes formed by non-segregating or segregating a pair of homologous chromosomes in advance during meiosis phase, will generate a variety of aneuploidy cells when the above-mentioned gamete combines each other or combines with a normal gamete. In addition, the aneuploidy cells will also generate during somatic cell division, such as a tumor cell having a high aberration rate, *etc.*

## Example

Unless otherwise stated, the following nucleic acids are used in the example:

the first strand of the first adaptor comprises a sequence of 5' AAGTCGGAGGCCAAGCGGTCGT 3',
the second strand of the first adaptor comprises a sequence of 5' TTGGCCTCCGACT 3',
the first strand of the second adaptor comprises a sequence of 5' GTCTCCAGTCGAAGCCCGACG 3',
the second strand of the second adaptor comprises a sequence of 5' GCTTCGACTGGAGA 3',
the first single-stranded DNA comprises a sequence of 5'TCCTAAGACCGCTTGGCCTCCG3', and the second single-stranded DNA comprises a sequence of 5'AGACAAGCTC$(N)_m$GATCGGGCTTCGACGGAG3', wherein motif $(N)_m$ represents an index sequence comprising m nucleotides, m is any integer ranging from 4nt to 10nt, N=A, T, G or C and the single-stranded nucleic acid for the cyclization step comprises 5 'TCGAGCTTGTCTTCCTAAGACCGC3'.

1. DNA Sample Collection

(1) 2ml peripheral blood was collected from 36 pregnant women, and then centrifuged at 1600g at 4°C for 10 minutes. Red blood cells were removed, and the remaining blood plasma was centrifuged at 16000g at 4°C for 10mins to remove the white cells. Plasma DNA was extracted from the plasma by using a SnoMag™ Circulating DNA Kit (SNOVA), and the resulting DNA was dissolved in a 50 μl TE solution.

2. Preparation of the DNA Library for sequencing

2. 1 rSAPtreatment:

The resulting DNA sample was treated by a rSAP enzyme in a reaction mixture comprising:

| | |
|---|---|
| DNA | 50μl |
| rSAP (1U/μl) | 6μl |
| 10X NE Buffer2 | 6μl |

The reaction was carried out in a PCR machine, and the process was set as: 37°C/45min, 65°C/10min, decreasing at a rate of 0.1°C/s to 4°C.

2.2 End-repairing:

The product of 2.1 was end-repaired in a reaction mixture comprising:

| | |
|---|---|
| Water | 11μl |
| 0.1M ATP | 2μl |
| 25mM dNTP | 2μl |
| T4 DNA polymerase (3U/μl) | 3μl |

The reaction procedure was set as: 12°C/20min, held at 4°C.

2.3 Litigating adaptors

Two adaptors were litigated to the product of 2.2 in a reaction mixture comprising

| | |
|---|---|
| DNA | 30μl |
| 10uM first adaptor | 3μl |
| 10uM second adaptor | 3μl |
| 2x rapid ligation buffer | 40μl |
| T4 DNA ligase (600U/ul) | 4μl |

The reaction was carried out in a PCR machine, and the process was set as: 20°C/30min, held at 4°C.

2.4 Strand-replacement and nick translation :

The product of 2.3 was subjected to strand replacement and nick translation by Mixing the product of 2.3 as following:

| | |
|---|---|
| DNA | 40μl |
| 10x taq buffer | 8μl |
| 0.1M ATP | 2μl |
| 25mM dNTP | 2μl |
| first single-stranded DNA | 2μl |
| second single-stranded DNA | 2μl |

The mixture obtained was placed in a PCR machine, and the process was set as 60°C /5min , decreased to 37°C and then the following were added to the mixture:

| | |
|---|---|
| T4 DNA ligase (600U/μl) | 2μl |
| Taq polymerase (5U/μl) | 2μl |

The mixture obtained was placed in a PCR machine, and the process was set as 37°C for 1 hour.

2.5 PCR amplification

The product of 2.4 was amplified in a reaction comprising:

| | |
|---|---|
| DNA | 45μL |
| 2xPfuCx Mix | 50μL |
| PfuCx Turbo polymerase | 3μL |
| first primer | 2.5μL |

(continued)

| | |
|---|---|
| second primer | 2.5μL |

And the process of the PCR was set as:

| | | |
|---|---|---|
| 94℃ | 2min | |
| 94℃ | 15s | |
| 56℃ | 30 s | 13 Cycles |
| 72℃ | 30 s | |
| 72℃ | 10 min | |
| 4℃ | Hold | |

The PCR product was used to carry out the following.

2.6 Isolating single strand

The PCR product was mixed with 30μL MyOne C1 streptavidin beads in a 1.5 ml tube. The tube was then placed in a magnetic grate until the solution was clear and the supernatant was removed. 30μL 1x bead binding buffer (BBB) was added to the tube which was placed in a magnetic grate again. This was repeated 3 times, and the supernatant was removed. 30μL 1x bead binding buffer and 5μL 0.5% Tween20 were added.

The PCR product was mixed with TE to a final volume of 60 μl, and 30 μl 1X bead binding buffer was then added.

80μl of obtained sample was added and then the resulting mixture was transferred to a tube containing MyOne C1 streptavidin beads, and the tube was stood for 15min after mixing.

The tube was placed in a magnetic grate until the solution was clear and the supernatant was removed.

100OμL 1x bead washing buffer (BWB) was added to the tube followed by mixing the content of the tube. The tube was placed in a magnetic grate until the solution was clear and the supernatant was removed.

78μL 0.1M NaOH was added to dissolve the magnetic beads.

The tube was placed in a magnetic grate until the solution was clear and the supernatant was transferred to a new 1.5ml EP tube.

37.5μL 0.3M MOPS acid was added to the new 1.5 ml EP tube.

2.7 Cyclization

Cyclization was performed at 37°C for 1.5h in a reaction mixture comprising:

| | |
|---|---|
| DNA | 112μl |
| Water | 178.3μl |
| 10x TA buffer | 35μl |
| 0.1M ATP | 3.5μl |

single strand nucleic acid (20μM) 20μl

| | |
|---|---|
| T4 DNA Ligase | 1.2μl |
| 37°C | 1.5h. |

3. Sequencing

The cyclic single strand DNA library was used to prepare a DNA nanoball according to an instruction of Complete Genomics Inc., and then was sequenced in a CG machine. Sequencing reads were obtained with one end reads being 19bp and the other end reads being 12bp.

4. Data analysis

Data analysis was performed according to the following steps:

Aligning the sequencing result obtained with a reference human genome sequence to obtain an aligned reads group;
Determining the total number of reads contained in the aligned reads group, to obtain a value of M;
based on the aligned reads group, determining the number of reads originating from a chromosome i, to obtain a value of $N_i$, wherein i represent a chromosome number;
based on the values M and $N_i$, determining a parameter representing relative percentage of chromosome i, to obtain a parameter R; and
based on the parameter R, determining whether a fetal genetic abnormality is present for the chromosome i.

The parameter R is the ratio of $N_i/M$. The inventors surprisingly found that by using this technical feature, the efficiency of detecting a chromosomal aneuploidy will be significantly improved.

The step of determining whether a fetal genetic abnormality is present for the chromosome i includes: obtaining a Z value based on equation of

$$Z_i = (R_{i,j} - mean_i) / sd_i,$$

wherein,

$$mean_i = \frac{1}{n} \sum_{j=1}^{n} R_{i,j},$$

$$sd_i = \sqrt{\frac{1}{n-1} (R_{i,j} - mean_i)^2}$$

j represents sample number, n represents the total number of the samples; and
comparing the Z value with a first preset value and a second preset value. The first preset value and second preset value are determined using a plurality of known samples. The first preset value is -3, and the second preset value is at least +3. A Z value of less than the first preset value is an indication of the presence of an additional chromosome i in the sample, and a Z value of greater than the second preset value is an indication of the absence of one chromosome i in the sample.

The results of the 36 samples analyzed are summarized in the following table:

| Sample No. | Result of karyotype analysis | CG sequencing result |
|---|---|---|
| S1 | Trisome 13 | Trisome 13 |
| S2 | Trisome 13 | Trisome 13 |
| S3 | normal | normal |
| S4 | normal | normal |
| S5 | normal | normal |
| S6 | normal | normal |
| S7 | normal | normal |
| S8 | normal | normal |
| S9 | normal | normal |
| S10 | normal | normal |

(continued)

| Sample No. | Result of karyotype analysis | CG sequencing result |
| --- | --- | --- |
| S11 | normal | normal |
| S12 | normal | normal |
| S13 | normal | normal |
| S14 | normal | normal |
| S15 | normal | normal |
| S16 | normal | normal |
| S17 | normal | normal |
| S18 | normal | normal |
| S19 | normal | normal |
| S20 | normal | normal |
| S21 | normal | normal |
| S22 | normal | normal |
| S23 | normal | normal |
| S24 | normal | normal |
| S25 | normal | normal |
| S26 | normal | normal |
| S27 | normal | normal |
| S28 | normal | normal |
| S29 | normal | normal |
| S30 | normal | normal |
| S31 | normal | normal |
| S32 | Trisome 21 | Trisome 21 |
| S33 | normal | normal |
| S34 | Trisome 18 | Trisome 18 |
| S35 | Trisome 21 | Trisome 21 |
| S36 | Trisome 18 | Trisome 18 |

Reference throughout this specification to "an embodiment," "some embodiments," "one embodiment", "another example," "an example," "a specific example," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example," "in an example," "in a specific example," or "in some examples," in various places throughout this specification are not necessarily referring to the same embodiment or example of the present disclosure. Furthermore, the particular features, structures, materials, or characteristics may be combined in any suitable manner in one or more embodiments or examples.

**Claims**

1. A method of preparing a library for sequencing using a DNA sample from a blood sample, comprising:

(a) isolating said DNA sample from the blood sample;
(b) dephosphorylating the DNA sample to obtain a dephosphorylated fragmentation product and

(c) subjecting dephosphorylated DNA fragments of said fragmentation product to the following steps to obtain a library of cyclic DNAs for sequencing:

(i) end-repairing to obtain a double-stranded DNA fragment having two blunt ends with four terminal nucleotides each lacking a phosphate group;

(ii) ligating a first adaptor and a second adaptor being different from each other with the double-stranded DNA fragment respectively at the two blunt ends in a one-step reaction to obtain a first ligation product, with each of the first adaptor and the second adaptor being an isolated oligonucleotide comprising: a first strand having a phosphate group at the 5'-terminus and a dideoxynucleotide at the 3'-terminus and a second strand lacking a phosphate group at the 5'-terminus and having a dideoxynucleotide at the 3'-terminus, said first strand having a length greater than that of said second strand and the first and second strands being formed into a double-stranded structure,

(iii) replacing the second strand of the first adaptor with a first single-stranded DNA and replacing the second strand of the second adaptor with a second single-stranded DNA, followed by a nick translation reaction thereby obtaining a second ligation product, with the first single-stranded DNA being adapted to form a double-stranded structure with the first strand of the first adaptor, and the second single-stranded DNA being adapted to form a double-stranded structure with the first strand of the second adaptor,

(iv) amplifying the second ligation product using a first primer and a second primer to obtain an amplified double-stranded DNA fragment, wherein the first primer comprises a sequence identical to one of said first single-stranded DNA and said second single-stranded DNA and the second primer comprises a sequence identical to the other of said first single-stranded DNA and said second single-stranded DNA and possesses an additional biotin;

(v) isolating a single-stranded DNA fragment containing biotin from said amplified double-stranded DNA fragment and

(vi) cyclizing the isolated single-stranded DNA fragment.

2. The method of claim 1, wherein the blood sample is a plasma sample,
said DNA sample is a circulating nucleic acid sample and
DNA fragments with a length of about 100 bp to 200 bp are provided for dephosphorylation.

3. The method of claim 1 or 2, wherein each of the first and second adaptors independently comprises:

a first overhang end located on the 3'-end of the first strand, and
an optional second overhang end located on the 5'-end of the first strand, optionally, in each of the first and second adaptors, the first overhang end has a length greater than that of the second overhang end,
preferably, in each of the first and second adaptors, the length of the first overhang end is about 6nt to 12nt,
preferably, in each of the first and second adaptors, the length of the second overhang end is about 0nt to 4nt.

4. The method of any one of claims 1 to 3, wherein in each of the first and second adaptors, the length of the first strand is about 20nt to 25nt,
optionally, the length of the second strand is about 10nt to 15nt,
optionally,
the double-stranded structure formed by the first single-stranded DNA and the first strand of the first adaptor has a length greater than that of the double-stranded structure formed by the first strand and the second strand in the first adaptor; and
the double-stranded structure formed by the second single-stranded DNA and the first strand of the second adaptor has a length greater than that of the double-stranded structure formed by the first strand and the second strand in the second adaptor,
optionally,
the first strand of the first adaptor comprises a sequence of 5'AAGTCGGAGGCCAAGCGGTCGT 3';
the second strand of the first adaptor comprises a sequence of 5' TTGGCCTCCGACT 3',
the first strand of the second adaptor comprises a sequence of 5' GTCTCCAGTCGAAGCCCGACG 3';
the second strand of the second adaptor comprises a sequence of 5' GCTTCGACTGGAGA 3',
the first single-stranded DNA comprises a sequence of 5 'TCCTAAGACCGCTTGGCCTCCG3', and the second single-stranded DNA comprises a sequence of 5'AGACAAGCTC(N)$_m$GATCGGGCTTCGACTGGAG3', wherein motif (N)$_m$ represents an index sequence comprising m nucleotides, m is any integer ranging from 4nt to 10 nt, N=A, T, G or C.

**5.** The method of any one of claims 1 to 4, wherein the step of replacing the second strand of the first adaptor with the first single-stranded DNA and replacing the second strand of the second adaptor with the second single-stranded DNA is performed by means of thermal lysis and annealing,

preferably, the thermal lysis is performed at a temperature of about 60 degrees Celsius.

**6.** The method of claim any one of claims 1 to 5, wherein the step of isolating the single-stranded DNA fragment containing biotin from the amplified double- stranded_DNA fragment further comprises the steps of

contacting the amplified double-stranded DNA fragment with a magnetic bead to form a bead-DNA composite, with the magnetic bead having a streptavidin linked thereto, and

contacting the bead-DNA composite with a solution having a pH greater than 7 to obtain the single-stranded DNA fragment containing biotin;

preferably, the solution having a pH greater than 7 comprising NaOH,
more preferably, the concentration of NaOH in the solution being about 0.5 to 2M, even more preferably about 1M;

optionally, the method further comprising the step of screening the amplified double-stranded DNA fragment prior to the step of isolating the single-stranded DNA fragment containing biotin,

preferably, the screening is performed by contacting the amplified double-stranded DNA fragment with a probe specific to a predetermined sequence,
more preferably, the predetermined sequence comprises at least one exon,
more preferably, the probe is provided in the form of a microarray.

**7.** The method of any one of claims 1 to 6, wherein the step of cyclizing the isolated_ single-stranded DNA fragment containing biotin is performed by using a single-stranded nucleic acid molecule comprising:

a first section adapted to match with either one of a sequence comprising the 5'-terminus nucleotide or a sequence comprising the 3'-terminus nucleotide in said isolated single- stranded DNA fragment containing biotin, and
a second section adapted to match with the other of a sequence comprising the 5'-terminus nucleotide or the 3'-terminus nucleotide in said isolated single-stranded DNA fragment containing biotin,
preferably, the first section and the second section being adjacently linked together,
preferably, said single-stranded nucleic acid molecule comprising the sequence 5'TCGAGCTTGTCTTCCTAAGACCGC 3'.

**8.** A method of sequencing a nucleic acid, comprising the steps of:

preparing a library for sequencing containing said nucleic acid according to the method of any one of claims 1 to 7, and
sequencing the library,
preferably the step of sequencing being performed by using a CG sequencing platform.

**9.** A method for determining a fetal genetic abnormality which is a chromosomal aneuploidy in a blood sample obtained from a pregnant female subject, comprising:

(a) sequencing at least a portion of a plurality of the nucleic acid molecules contained in the blood sample to obtain a sequencing result according to claim 8;
(b) aligning the sequencing result obtained in step (a) with a reference human genome sequence, to obtain an aligned reads group;
(c) determining the total number of reads contained in the aligned reads group obtained in step (b), to obtain a value of M;
(d) based on the aligned reads group obtained in step (b), determining the number of reads originating from a chromosome i, to obtain a value of $N_i$, wherein i represent a chromosome number;
(e) based on the values M and $N_i$, determining a parameter representing relative percentage of chromosome i, to obtain a parameter R; and
(f) based on the parameter R, determining whether a fetal genetic abnormality is present for the chromosome i,

wherein the parameter R is the ratio of $N_i/M$.

**10.** The method of claim 9, wherein the read has a length ranging from about 12bp to 21 bp.

**11.** The method of claim 9 or 10, wherein the aligned reads group consists of uniquely aligned reads.

**12.** The method of any one of claims 9 to 11, wherein step (f) further comprises:

obtaining a Z value based on the equation of

$$Z_i = (R_{i,j} - mean_i) / sd_i,$$

wherein,

$$mean_i = \frac{1}{n} \sum_{j=1}^{n} R_{i,j},$$

$$sd_i = \sqrt{\frac{1}{n-1}(R_{i,j} - mean_i)^2}$$

j represents sample number, n represents the total number of the samples; and
comparing the Z value with a first preset value and a second preset value,

preferably, the first preset value and the second preset value are determined using a plurality of known samples,
more preferably, the first preset value is no more than -3,
even more preferably, the second preset value is at least +3,
even more preferably, the Z value of less than the first preset value is an indication of the presence of additional one chromosome i in the sample, and the Z value of greater than the second preset value is an indication of the absence of one chromosome i in the sample.

**13.** An apparatus to prepare a library for sequencing as claimed in claim 1, comprising:

(a) a DNA-sample-isolating unit adapted to isolate a DNA sample from the blood sample;
(b) a dephosphorylation unit adapted to dephosphorylate the DNA sample to obtain a dephosphorylated fragmentation product;
(c) an end-repairing unit adapted to carry out said end-repairing step;
(d) a first ligation unit adapted to carry out said ligating step to obtain said first ligation product;
(e) a strand-replacing and nick-translating unit adapted to replace the second strand of the first adaptor with a first single-stranded DNA and replace the second strand of the second adaptor with a second single-stranded DNA followed by a nick translation reaction thereby obtaining said second ligation product;
(f) an amplification unit adapted to amplify the second ligation product to obtain said amplified double-stranded DNA fragment;
(g) a single-strand-DNA-isolating unit adapted to isolate said single-stranded DNA fragment containing biotin from said amplified double-stranded DNA fragment and
(h) a cyclizing unit adapted to cyclize the isolated single-stranded DNA fragment,

whereby a library of cyclic DNAs can be formed for sequencing.

**14.** An assembly to sequence a nucleic acid, comprising:

an apparatus to prepare a library for sequencing according to claim 13, and
a sequencing apparatus adapted to sequence the library,
preferably the sequencing apparatus being a CG sequencing platform.

**15.** A system for determining a fetal genetic abnormality which is a chromosomal aneuploidy in a blood sample obtained from a pregnant female subject, comprising:

an assembly according to claim 14 adapted to sequence at least a portion of a plurality of the nucleic acid molecules contained in said sample to obtain a sequencing result;
an assembly adapted to analyse the sequencing result according to the following steps of:

aligning the sequencing result with a reference human genome sequence to obtain an aligned reads group;
determining the total number of reads contained in the aligned reads group to obtain a value of M;
based on the aligned reads group, determining the number of reads originating from a chromosome i, to obtain a value of $N_i$, wherein i represent a chromosome number;
based on the values M and $N_i$, determining a parameter representing a relative percentage of chromosome i, to obtain a parameter R; and
based on the parameter R, determining whether the fetal genetic abnormality is present for the chromosome i,

preferably the parameter R is the ratio of $N_i/M$.

**Patentansprüche**

1.  Verfahren zum Aufbau einer Bibliothek zur Sequenzierung unter Verwendung einer DNA-Probe aus einer Blutprobe, wobei das Verfahren folgendes umfasst:

    (a) Isolieren der DNA-Probe aus der Blutprobe;
    (b) Dephosphorylieren der DNA-Probe, so dass ein dephosphoryliertes Fragmentierungsprodukt erhalten wird; und
    (c) Unterziehen von dephosphorylierten DNA-Fragmenten des Fragmentierungsprodukts den folgenden Schritten, um eine Bibliothek cyclischer DNA zur Sequenzierung zu erhalten:

    (i) Endreparatur, um ein doppelsträngiges DNA-Fragment mit zwei stumpfen Enden mit vier terminalen Nucleotiden zu erhalten, denen jeweils eine Phosphatgruppe fehlt;
    (ii) Ligieren eines ersten Adapters und eines zweiten Adapters, die sich voneinander unterscheiden, mit dem doppelsträngigen DNA-Fragment an den entsprechenden beiden stumpfen Enden in einer Einschrittreaktion, um ein erstes Ligationsprodukt zu erhalten, wobei der erste Adapter und der zweite Adapter jeweils ein isoliertes Oligonucleotid ist, umfassend: einen ersten Strang mi einer Phosphatgruppe an dem 5'-Ende und einem Dideoxynucleotid an dem 3'-Ende und einen zweiten Strang, dem eine Phosphatgruppe an dem 5'-Ende fehlt und der ein Dideoxynucleotid an dem 3'-Ende aufweist, wobei der erste Strang länger ist als der zweite Strang, und wobei der erste und der zweite Strang in eine doppelsträngige Struktur gebildet werden;
    (iii) Ersetzen des zweiten Strangs des ersten Adapters mit einer ersten einsträngigen DNA und Ersetzen des zweiten Strangs des zweiten Adapters mit einer zweiten einsträngigen DNA, gefolgt von einer Nick Translations-Reaktion, wodurch ein zweites Ligationsprodukt erhalten wird, wobei die erste einsträngige DNA eine doppelsträngige Struktur mit dem ersten Strang des ersten Adapters bilden kann, und wobei die zweite einsträngige DNA eine doppelsträngige Struktur mit dem ersten Strang des zweiten Adapters bilden kann;
    (iv) Amplifizieren des zweiten Ligationsprodukts unter Verwendung eines ersten Primers und eines zweiten Primers, um ein amplifiziertes doppelsträngiges DNA-Fragment zu erhalten, wobei der erste Primer eine Sequenz umfasst, die identisch ist mit der ersten einsträngigen DNA oder der zweiten einsträngigen DNA, und wobei der zweite Primer eine Sequenz umfasst, die identisch ist der anderen der ersten einsträngigen DNA oder der zweiten einsträngigen DNA und ein zusätzliches Biotin besitzt;
    (v) Isolieren eines einsträngigen DNA-Fragments, das Biotin enthält, aus dem amplifizierten doppelsträngigen DNA-Fragment; und
    (vi) Cyclisieren des isolierten einsträngigen DNA-Fragments.

2.  Verfahren nach Anspruch 1, wobei die Blutprobe eine Plasmaprobe ist,
    wobei die DNA-Probe eine zirkulierende Nucleinsäureprobe ist, und wobei DNA-Fragmente mit einer Länge von etwa 100 bp bis 200 bp zur Dephosphorylierung bereitgestellt werden.

3.  Verfahren nach Anspruch 1 oder 2, wobei der erste und zweite Adapter jeweils unabhängig folgendes umfassen:

    ein erstes Überhangende, das sich an dem 3'-Ende des ersten Strangs befindet;

ein optionales zweites Übergangende, das sich an dem 5'-Ende des ersten Strangs befindet;
wobei das erste Überhangende in dem ersten und zweiten Adapter jeweils eine Länge aufweist, die größer ist als die Länge des zweiten Überhangendes;
wobei vorzugsweise in dem ersten und zweiten Adapter jeweils die Länge des ersten Überhangendes jeweils etwa 6 nt bis 12 nt beträgt;
wobei vorzugsweise in dem ersten und zweiten Adapter jeweils die Länge des zweiten Überhangendes jeweils etwa 0 nt bis 4 nt beträgt.

4. Verfahren nach einem der Ansprüche 1 bis 3, wobei die Länge des ersten Strangs in dem ersten und zweiten Adapter jeweils etwa 20 nt bis 25 nt beträgt;
wobei die Länge des zweiten Strangs optional etwa 10 nt bis 15 nt beträgt;
wobei optional die doppelsträngige Struktur, die durch die erste einsträngige DNA und den ersten Strang des ersten Adapters gebildet wird, eine Länge aufweist, die größer ist als die Länge der durch den ersten Strang und den zweiten Strang in dem ersten Adapter gebildete doppelsträngigen Struktur; und
die durch die zweite einsträngige DNA und den ersten Strang des zweiten Adapters gebildete doppelsträngige Struktur eine Länge aufweist, die größer ist als die Länge der durch den ersten Strang und den zweiten Strang in dem zweiten Adapter gebildete doppelsträngige Struktur;
wobei optional der erste Strang des ersten Adapters eine Sequenz 5'AAGTCGGAGGCCAAGCGGTCGT3' umfasst;
der zweite Strang des ersten Adapters eine Sequenz 5'TTGGCCTCCGACT3' umfasst;
der erste Strang des zweiten Adapters eine Sequenz 5'GTCTCCAGTCGAAGCCCGACG3' umfasst;
der zweite Strang des zweiten Adapters eine Sequenz 5'TCCTAAGACCGCTTGGCCTCCG3' umfasst; und
die zweite einsträngige DNA eine Sequenz 5'AGACAAGCTC(N)$_m$GATCGGGCTTCGACTGGAG3' umfasst, wobei das Motiv (N)$_m$ eine Indexsequenz darstellt, die m Nucleotide umfasst, wobei m eine ganze Zahl zwischen 4 nt und 10 nt ist, mit N=A, T, G oder C.

5. Verfahren nach einem der Ansprüche 1 bis 4, wobei der Schritt des Ersetzens des zweiten Strangs des ersten Adapters mit der ersten einsträngigen DNA und des zweiten Strangs des zweiten Adapters mit der zweiten einsträngigen DNA durch Thermolyse und Tempern durchgeführt wird;
wobei die Thermolyse vorzugsweise bei einer Temperatur von etwa 60 Grad Celsius durchgeführt wird.

6. Verfahren nach einem der Ansprüche 1 bis 5, wobei der Schritt des Isolierens des einsträngigen DNA-Fragments, das Biotin enthält, aus dem amplifizierten doppelsträngigen DNA-Fragment ferner die folgenden Schritte umfasst:

Inkontaktbringen des amplifizierten doppelsträngigen DNA-Fragments mit einem Magnetic Bead, um eine Bead-DNA-Verbundstruktur zu bilden, wobei das Magnetic Bead damit gekoppeltes Streptavidin aufweist; und
Inkontaktbringen der Bead-DNA-Verbundstruktur mit einer Lösung mit einem pH-Wert von mehr als 7, um das einsträngige DNA-Fragment zu erhalten, das Biotin enthält;

wobei vorzugsweise die Lösung, die einen pH-Wert von mehr als 7 aufweist, NaOH umfasst;
wobei darüber hinaus bevorzugt die Konzentration von NaOH in der Lösung etwa 0,5 bis 2 M entspricht, darüber hinaus bevorzugt etwa 1 M;

wobei das Verfahren optional ferner den Schritt des Screenings des amplifizierten doppelsträngigen DNA-Fragments vor dem Schritt des Isolierens des einsträngigen DNA-Fragments, das Biotin enthält, umfasst;

wobei das Screening vorzugsweise durchgeführt wird durch Inkontaktbringen des amplifizierten doppelsträngigen DNA-Fragments mit einer für eine vorbestimmte Sequenz spezifischen Probe;
wobei darüber hinaus bevorzugt die vorbestimmte Sequenz wenigstens ein Exon umfasst,
wobei darüber hinaus bevorzugt die Probe in Form eines Microarrays bereitgestellt wird.

7. Verfahren nach einem der Ansprüche 1 bis 6, wobei der Schritt des Cyclisierens des isolierten einsträngigen DNA-Fragments, das Biotin enthält, durchgeführt wird unter Verwendung eines einsträngigen Nucleinsäuremoleküls, das folgendes umfasst:

einen ersten Abschnitt, der geeignet ist für ein Matching mit einer Sequenz, die das 5'-Ende-Nucleotid umfasst, oder mit einer Sequenz, die das 3'-Ende-Nucleotid umfasst, in dem isolierten einsträngigen DNA-Fragment, das Biotin enthält; und
einen zweiten Abschnitt, der geeignet ist für ein Matching mit der anderen Sequenz der Sequenz, die das 5'-Ende-Nucleotid umfasst, oder die das 3'-Ende-Nucleotid umfasst, in dem isolierten einsträngigen DNA-Fragment, das Biotin enthält;
wobei vorzugsweise der erste Abschnitt und der zweite Abschnitt benachbart und miteinander gekoppelt sind;
wobei vorzugsweise das einsträngige Nucleinsäuremolekül die Sequenz 5'TCGAGCTTGTCTTCCTAAGACCGC3' umfasst.

8. Verfahren zur Sequenzierung einer Nucleinsäure, welches die folgenden Schritte umfasst:

Aufbauen einer Bibliothek zur Sequenzierung, die Nucleinsäure enthaltend, nach dem Verfahren nach einem der Ansprüche 1 bis 7; und
Sequenzieren der Bibliothek;
wobei vorzugsweise der Schritt des Sequenzierens unter Verwendung einer CG-Sequenzierungsplattform durchgeführt wird.

9. Verfahren zur Bestimmung einer fetalen genetischen Anomalität, bei der es sich um eine chromosomale Aneuploidie in einer Blutprobe von einem schwangeren weiblichen Subjekt handelt, wobei das Verfahren folgendes umfasst:

(a) Sequenzieren wenigstens eines Teils mehrerer, in der Blutprobe enthaltener Nucleinsäuremoleküle, um ein Sequenzierungsergebnis nach Anspruch 8 zu erhalten;
(b) Alignment des in Schritt (a) erhaltenen Sequenzierungsergebnisses mit einer menschlichen Referenzgenomsequenz, um eine alignierte Reads-Gruppe zu erhalten;
(c) Bestimmen einer Gesamtanzahl enthaltener Reads in der in Schritt (b) erhaltenen alignierten Reads-Gruppe, um einen Wert von M zu erhalten;
(d) auf der Basis der in Schritt (b) erhaltenen Reads-Gruppe, Bestimmen der von einem Chromosom i stammenden Anzahl von Reads, um einen Wert von $N_i$ zu erhalten, wobei i eine Chromosomenzahl darstellt;
(e) auf der Basis der Werte M und $N_i$, Bestimmen eines Parameters, der den relativen prozentualen Anteil von Chromosom i darstellt, um einen Parameter R zu erhalten; und
(f) basierend auf dem Parameter R, Bestimmen, ob für das Chromosom i eine fetale genetische Anomalität vorliegt,

wobei der Parameter R das Verhältnis von $N_i/M$ ist.

10. Verfahren nach Anspruch 9, wobei der Read eine Länge zwischen 12 bp und 21 bp aufweist.

11. Verfahren nach Anspruch 9 oder 10, wobei sie alignierte Reads-Gruppe aus eindeutig alignierten Reads besteht.

12. Verfahren nach einem der Ansprüche 9 bis 11, wobei Schritt (f) ferner folgendes umfasst:

Ermitteln eines Wertes von Z auf der Basis der Gleichung

$$Z_i = (R_{i,j} - mean_i) / sd_i,$$

wobei:

$$mean_i = \frac{1}{n} \sum_{j=1}^{n} R_{i,j},$$

$$sd_i = \sqrt{\frac{1}{n-1} (R_{i,j} - mean_i)^2}$$

j eine Probennummer darstellt, n die Anzahl der Proben insgesamt darstellt; und
Vergleichen des Wertes von Z mit einem ersten voreingestellten Wert und einem zweiten voreingestellten Wert;

wobei vorzugsweise der erste voreingestellte Wert und der zweite voreingestellte Wert bestimmt werden unter Verwendung einer Mehrzahl bekannter Proben;

wobei darüber hinaus bevorzugt der erste voreingestellte Wert nicht größer ist als -3;

wobei wiederum darüber hinaus bevorzugt der zweite voreingestellte Wert mindestens +3 beträgt;

wobei wiederum darüber hinaus bevorzugt ein Wert von Z, der kleiner ist als der erste voreingestellte Wert, eine Indikation für das Vorhandensein des zusätzlichen einen Chromosoms i in der Probe ist, und ein Wert von Z, der größer ist als der zweite voreingestellte Wert, eine Indikation für das Fehlen eines Chromosoms i in der Probe ist.

13. Vorrichtung zum Aufbau einer Bibliothek zur Sequenzierung nach Anspruch 1, wobei die Vorrichtung folgendes umfasst:

(a) eine DNA-Proben-Isolationseinheit zum Isolieren einer DNA-Probe aus der Blutprobe;

(b) eine Dephosphorylierungseinheit, die geeignet ist zum Dephosphorylieren der DNA-Probe, so dass ein dephosphoryliertes Fragmentierungsprodukt erhalten wird;

(c) eine Endreparatureinheit, die geeignet ist zum Ausführen des Endreparaturschrittes;

(d) eine erste Ligationseinheit, die sich zur Ausführung des Schrittes des Ligierens eignet, um ein erstes Ligationsprodukt zu erhalten;

(e) eine Strangersatz- und Nick Translations-Einheit, die geeignet ist zum Ersetzen des zweiten Strangs des ersten Adapters mit einer ersten einsträngigen DNA und Ersetzen des zweiten Strangs des zweiten Adapters mit einer zweiten einsträngigen DNA, gefolgt von einer Nick Translations-Reaktion, wodurch ein zweites Ligationsprodukt erhalten wird;

(f) eine Amplifikationseinheit, die sich zum Amplifizieren des zweiten Ligationsprodukts eignet, um das amplifizierte doppelsträngige DNA-Fragment zu erhalten;

(g) eine Einzelstrang-DNA-Isolationseinheit, die geeignet ist zum Isolieren des einsträngigen DNA-Fragments, das Biotin enthält, aus dem amplifizierten doppelsträngigen DNA-Fragment; und

(h) eine Cyclisierungseinheit, die geeignet ist zum Cyclisieren des isolierten einsträngigen DNA-Fragments, wodurch eine Bibliothek cyclischer DNA zur Sequenzierung aufgebaut werden kann.

14. Anordnung zur Sequenzierung einer Nucleinsäure, wobei die Anordnung folgendes umfasst:

eine Vorrichtung zum Aufbau einer Bibliothek zur Sequenzierung nach Anspruch 13; und

eine Sequenzierungsvorrichtung, die sich zur Sequenzierung der Bibliothek eignet,

wobei die Sequenzierungsvorrichtung vorzugsweise eine CG-Sequenzierungsplattform ist.

15. System zur Bestimmung einer fetalen genetischen Anomalität, bei der es sich um eine chromosomale Aneuploidie in einer Blutprobe von einem schwangeren weiblichen Subjekt handelt, wobei das System folgendes umfasst:

eine Anordnung nach Anspruch 14, die geeignet ist zum Sequenzieren wenigstens eines Teils einer Mehrzahl von Nucleinsäuremolekülen, die in der Probe enthalten sind, um ein Sequenzierungsergebnis zu erhalten;

eine Anordnung, die geeignet ist, das Sequenzierungsergebnis gemäß den folgenden Schritten zu analysieren:

Alignieren des Sequenzierungsergebnisses mit einer menschlichen Referenzgenomsequenz, um eine alignierte Reads-Gruppe zu erhalten;

Bestimmen einer Gesamtanzahl der in der alignierten Reads-Gruppe enthaltenen Reads, um einen Wert von M zu erhalten;

auf der Basis der erhaltenen Reads-Gruppe, Bestimmen der von einem Chromosom i stammenden Anzahl von Reads, um einen Wert von $N_i$ zu erhalten, wobei i eine Chromosomenzahl darstellt;

auf der Basis der Werte M und $N_i$, Bestimmen eines Parameters, der den relativen prozentualen Anteil von Chromosom i darstellt, um einen Parameter R zu erhalten; und

basierend auf dem Parameter R, Bestimmen, ob für das Chromosom i eine fetale genetische Anomalität vorliegt,

wobei der Parameter R das Verhältnis von $N_i/M$ ist.

**Revendications**

1. Procédé de préparation d'une banque pour le séquençage à l'aide d'un échantillon d'ADN provenant d'un échantillon de sang, comprenant les étapes consistant à :

(a) isoler ledit échantillon d'ADN de l'échantillon de sang ;
(b) déphosphoryler l'échantillon d'ADN pour obtenir un produit de fragmentation déphosphorylé, et
(c) soumettre les fragments d'ADN déphosphorylés dudit produit de fragmentation aux étapes suivantes pour obtenir une banque d'ADN cycliques pour le séquençage:

(i) réparer en bout pour obtenir un fragment d'ADN double brin ayant deux extrémités franches avec quatre nucléotides terminaux dépourvus chacun d'un groupe phosphate ;
(ii) ligaturer un premier adaptateur et un second adaptateur différents l'un de l'autre avec le fragment d'ADN double brin respectivement aux deux extrémités franches dans une réaction en une étape pour obtenir un premier produit de ligature, chacun du premier adaptateur et du second adaptateur étant un oligonucléotide isolé comprenant : un premier brin ayant un groupe phosphate à l'extrémité 5' et un dideoxynucléotide à l'extrémité 3' et un second brin dépourvu d'un groupe phosphate à l'extrémité 5' et ayant un dideoxynucléotide à l'extrémité 3', ledit premier brin ayant une longueur supérieure à celle dudit second brin et les premier et second brins étant formés en une structure double brin,
(iii) remplacer le second brin du premier adaptateur par un premier ADN simple brin et remplacer le second brin du second adaptateur par un second ADN simple brin, suivi d'une réaction de translation de coupure, obtenant ainsi un second produit de ligature, le premier ADN simple brin étant conçu pour former une structure double brin avec le premier brin du premier adaptateur, et le second ADN simple brin étant conçu pour former une structure double brin avec le premier brin du second adaptateur,
(iv) amplifier le second produit de ligature à l'aide d'une première amorce et d'une seconde amorce pour obtenir un fragment d'ADN double brin amplifié, la première amorce comprenant une séquence identique à l'un dudit premier ADN simple brin et dudit second ADN simple brin et la seconde amorce comprenant une séquence identique à l'autre dudit premier ADN simple brin et dudit second ADN simple brin et ayant une biotine supplémentaire ;
(v) isoler un fragment d'ADN simple brin contenant de la biotine dudit fragment d'ADN double brin amplifié et
(vi) cycliser le fragment d'ADN simple brin isolé.

2. Procédé selon la revendication 1, l'échantillon de sang étant un échantillon de plasma, ledit échantillon d'ADN étant un échantillon d'acide nucléique circulant et des fragments d'ADN d'une longueur d'environ 100 bp à 200 bp étant prévus pour déphosphorylation.

3. Procédé selon la revendication 1 ou 2, chacun des premier et second adaptateurs comprenant indépendamment :

une première extrémité en surplomb située sur l'extrémité 3' du premier brin, et
une seconde extrémité en surplomb optionnelle située sur l'extrémité 5' du premier brin,
éventuellement, dans chacun des premier et second adaptateurs, la première extrémité en surplomb ayant une longueur supérieure à celle de la seconde extrémité en surplomb,
de préférence, dans chacun des premier et second adaptateurs, la longueur de la première extrémité en surplomb étant d'environ 6 nt à 12 nt,
de préférence, dans chacun des premier et second adaptateurs, la longueur de la seconde extrémité en surplomb étant d'environ 0 nt à 4 nt.

4. Procédé selon l'une quelconque des revendications 1 à 3, dans chacun des premier et second adaptateurs, la longueur du premier brin étant d'environ 20 nt à 25 nt,
éventuellement, la longueur du second brin étant d'environ 10 nt à 15 nt,
éventuellement,
la structure double brin formée par le premier brin d'ADN simple brin et le premier brin du premier adaptateur ayant une longueur supérieure à celle de la structure double brin formée par le premier brin et le second brin dans le premier adaptateur ; et
la structure double brin formée par le second brin d'ADN simple brin et le premier brin du second adaptateur ayant une longueur supérieure à celle de la structure double brin formée par le premier brin et le second brin dans le second adaptateur,
éventuellement,
le premier brin du premier adaptateur comprenant une séquence de 5'AAGTCGGAGGCCAAGCGGTCGT 3' ;
le second brin du premier adaptateur comprenant une séquence de 5'TTGGCCTCCGACT 3',
le premier brin du second adaptateur comprenant une séquence de 5'GTCTCCAGTCGAAGCCCGACG 3' ;

le second brin du second adaptateur comprenant une séquence de 5'GCTTCGACTGGAGA 3' ;

le premier ADN simple brin comprenant une séquence de 5'TCCTAAGACCGCTTGGCCTCCG3',

et le second ADN simple brin comprenant une séquence de 5'AGACAAGCTC(N)$_m$GATCGGGCTTCGACTGGAG3',

le motif (N)$_m$ représentant une séquence d'index comprenant m nucléotides, m étant tout entier compris entre 4 nt et 10 nt, N=A, T, G ou C.

**5.** Procédé selon l'une quelconque des revendications 1 à 4, le remplacement du second brin du premier adaptateur par le premier ADN simple brin et le remplacement du second brin du second adaptateur par le second ADN simple brin étant effectuée au moyen d'une lyse thermique et d'un annelage,

de préférence, la lyse thermique étant effectuée à une température d'environ 60 degrés Celsius.

**6.** Procédé selon l'une quelconque des revendications 1 à 5, l'isolement du fragment d'ADN simple brin contenant de la biotine du fragment d'ADN double brin amplifié comprenant en outre les étapes consistant à :

mettre en contact le fragment d'ADN double brin amplifié avec une bille magnétique pour former un composite bille-ADN, la bille magnétique ayant une streptavidine liée à elle, et

mettre en contact le composite bille-ADN avec une solution ayant un pH supérieur à 7 pour obtenir le fragment d'ADN simple brin contenant de la biotine ;

de préférence, la solution ayant un pH supérieur à 7 comprenant du NaOH,
plus préférentiellement, la concentration de NaOH dans la solution étant d'environ 0,5 à 2 M,
encore plus préférentiellement autour de 1 M ;

éventuellement, le procédé comprenant en outre l'étape consistant à cribler le fragment d'ADN double brin amplifié avant l'isolement du fragment d'ADN simple brin contenant de la biotine,

de préférence, le criblage étant effectué en mettant en contact le fragment d'ADN double brin amplifié avec une sonde spécifique à une séquence prédéfinie,
plus préférentiellement, la séquence prédéfinie comprenant au moins un exon,
plus préférentiellement, la sonde étant fournie sous la forme d'un microréseau.

**7.** Procédé selon l'une quelconque des revendications 1 à 6, la cyclisation du fragment d'ADN simple brin isolé contenant de la biotine étant réalisée en utilisant une molécule d'acide nucléique simple brin comprenant :

une première section conçue pour correspondre à l'une ou l'autre d'une séquence comprenant le nucléotide à extrémité 5' ou d'une séquence comprenant le nucléotide à extrémité 3' dans ledit fragment d'ADN simple brin isolé contenant de la biotine, et

une seconde section conçue pour correspondre à l'autre d'une séquence comprenant le nucléotide à extrémité 5' ou le nucléotide à extrémité 3' dans ledit fragment d'ADN simple brin isolé contenant de la biotine,

de préférence, la première section et la seconde section étant adjacentes l'une à l'autre,

de préférence, ladite molécule d'acide nucléique simple brin comprenant la séquence 5'TCGAGCTTGTCTTCCTAAGACCGC 3'.

**8.** Procédé de séquençage d'un acide nucléique, comprenant les étapes consistant à :

préparer une banque de séquençage contenant ledit acide nucléique selon le procédé selon l'une quelconque des revendications 1 à 7, et

séquencer la banque,

de préférence, le séquençage étant réalisé à l'aide d'une plate-forme de séquençage CG.

**9.** Procédé pour déterminer une anomalie génétique foetale qui est une aneuploïdie chromosomique dans un échantillon de sang obtenu à partir d'une femelle enceinte sujet, comprenant les étapes consistant à :

(a) séquencer au moins une partie d'une pluralité de molécules d'acide nucléique contenues dans l'échantillon de sang pour obtenir un résultat de séquençage selon la revendication 8 ;

(b) aligner le résultat du séquençage obtenu à l'étape (a) sur une séquence de référence du génome humain, pour obtenir un groupe de lectures alignées ;

(c) déterminer le nombre total de lectures contenues dans le groupe de lectures alignées obtenu à l'étape (b), pour obtenir une valeur de M ;

(d) sur la base du groupe de lectures alignées obtenu à l'étape (b), déterminer le nombre de lectures provenant d'un chromosome i, pour obtenir une valeur de $N_i$, où i représente un nombre de chromosomes ;

(e) sur la base des valeurs M et $N_i$, déterminer un paramètre représentant un pourcentage relatif du chromosome i, pour obtenir un paramètre R ; et

(f) en fonction du paramètre R, déterminer si une anomalie génétique foetale est présente pour le chromosome i,

le paramètre R étant le rapport $N_i/M$.

10. Procédé selon la revendication 9, la lecture ayant une longueur comprise entre environ 12 bp et 21 bp.

11. Procédé selon la revendication 9 ou 10, le groupe de lectures alignées étant constitué de lectures alignées de façon unique.

12. Procédé selon l'une quelconque des revendications 9 à 11, l'étape (f) comprenant en outre les étapes consistant à :

obtenir une valeur Z en fonction de l'équation

$$Z_i = (R_{i,j} - mean_i) / sd_i,$$

où,

$$mean_i = \frac{1}{n} \sum_{j=1}^{n} R_{i,j},$$

$$sd_i = \sqrt{\frac{1}{n-1}(R_{i,j} - mean_i)^2}$$

j représente le nombre d'échantillons, n représente le nombre total des échantillons ; et
comparer la valeur Z avec une première valeur prédéfinie et une seconde valeur prédéfinie,

de préférence, la première valeur prédéfinie et la seconde valeur prédéfinie étant déterminées à l'aide d'une pluralité d'échantillons connus,
de préférence, la première valeur prédéfinie n'étant pas supérieure à -3,
de préférence encore, la seconde valeur prédéfinie étant d'au moins +3,
de préférence encore, la valeur Z inférieure à la première valeur prédéfinie étant une indication de la présence d'un chromosome i supplémentaire dans l'échantillon, et la valeur Z supérieure à la seconde valeur prédéfinie étant une indication de l'absence d'un chromosome i dans l'échantillon.

13. Appareil pour préparer une banque pour le séquençage selon la revendication 1, comprenant :

(a) une unité d'isolement d'échantillon d'ADN conçue pour isoler un échantillon d'ADN de l'échantillon de sang ;
(b) une unité de déphosphorylation conçue pour déphosphoryler l'échantillon d'ADN pour obtenir un produit de fragmentation déphosphorylé ;
(c) une unité de réparation d'extrémité conçue pour effectuer ladite étape de réparation d'extrémité ;
(d) une première unité de ligature conçue pour effectuer ladite étape de ligature pour obtenir ledit premier produit de ligature ;
(e) une unité de remplacement de brin et de translation de coupure conçue pour remplacer le second brin du premier adaptateur par un premier ADN simple brin et remplacer le second brin du second adaptateur par un second ADN simple brin suivi d'une réaction de translation de coupure, obtenant ainsi ledit second produit de ligature ;
(f) une unité d'amplification conçue pour amplifier le second produit de ligature pour obtenir ledit fragment d'ADN double brin amplifié ;
(g) une unité d'isolement d'ADN simple brin conçue pour isoler ledit fragment d'ADN simple brin contenant de la biotine à partir dudit fragment d'ADN double brin amplifié ; et

(h) une unité de cyclisation conçue pour cycliser le fragment d'ADN simple brin isolé,

moyennant quoi une banque d'ADN cycliques peut être formée pour le séquençage.

14. Ensemble pour séquencer un acide nucléique, comprenant :

un appareil pour préparer une banque pour le séquençage selon la revendication 13, et
un appareil de séquençage conçu pour séquencer la banque,
de préférence l'appareil de séquençage étant une plate-forme de séquençage CG.

15. Système pour déterminer une anomalie génétique foetale qui est une aneuploïdie chromosomique dans un échantillon de sang obtenu à partir d'une femelle enceinte sujet, comprenant :

un ensemble selon la revendication 14 conçu pour séquencer au moins une partie d'une pluralité de molécules d'acide nucléique contenues dans ledit échantillon pour obtenir un résultat de séquençage ;
un ensemble conçu pour analyser le résultat du séquençage selon les étapes consistant à :

aligner le résultat du séquençage sur une séquence de référence du génome humain, pour obtenir un groupe de lectures alignées ;
déterminer le nombre total de lectures contenues dans le groupe de lectures alignées pour obtenir une valeur de M ;

en fonction du groupe de lectures alignées, déterminer le nombre de lectures provenant d'un chromosome i, pour obtenir une valeur de $N_i$, où i représente un nombre de chromosomes ;
en fonction des valeurs M et $N_i$, déterminer un paramètre représentant un pourcentage relatif du chromosome i, pour obtenir un paramètre R ; et
en fonction du paramètre R, déterminer si l'anomalie génétique foetale est présente pour le chromosome i, de préférence le paramètre R étant le rapport $N_i/M$.